# EUROPEAN PATENT APPLICATION

(11) **EP 2 476 370 A1**
(43) Date of publication of application: **18.07.2012**
(21) Application number: 11151155.6
(22) Date of filing: 17.01.2011
(51) Int. Cl.: A61B 5/04

(54) **Electronic device and platform for collecting and transmitting electrical signals**

(71) Applicant: Catsanis, Nicholas, Sarjah (AE)
(72) Inventor: PETROVICH ROGOZENKO, Nicolai, 117534, Moscow (RU)
(74) Representative: Ström & Gulliksson AB

(57) **Abstract**

The present invention relates to a device and platform for collecting and transmitting electric signals to and from an end user, preferably having an amplitude below 0,1 mV. The device which is a part of the platform comprises a collector (2) and a processing device (4). The collector (2) is configured to detect and collect data of electric signals emitted by the end user and send such data to the processing device (4), which is configured to process data of the electrical signals in such a way that sets of pertinent data characteristics are obtainable and then correlate, compare and analyze the pertinent data characteristics with corresponding data characteristics stored in a reference database available via the communication network. The processing device is further configured to, in response to the correlation, comparison and analyzing, receive data, via the communication network (6), for generation of biocompatible electrical spectra, which data is sent to the collector.

## Description

### Technical field

The present invention relates to an electronic device and platform for collecting and transmitting electrical signals having a very low electrical nature, typically in the range of 0,1 mV and below. Such a device may especially be used for measuring the electric activity in a human body or outputting electrical activity into the human body.

### Background art

Today the costs for medical treatment are ever increasing. Mainly due to the fact that there is more and more advanced and expensive technical equipment at hand, which has made it possible for the health care sector to be more successful in treating different diseases. However, expensive equipment is not available anywhere and logistical factors like availability, operators and physical presence to use further drive the costs.

Some of these logistic problems are overcome by the use of an emerging health technology called telemedicine, The fundamental idea behind telemedicine is to collect basic medical data remotely at the location where the patient is. The collected medical data is converted and packed such that it may be electronically communicated and managed between patient and different medical service providers. The devices used by the patient contain similar technology as the professional medical devices mentioned above, but have a user-friendly interface and are connectable to a communication network. The great advantage with telemedicine is that it reduces the number of medical visits for a patient and improves the monitoring arid participation of the patient.

Furthermore, in telemedicine the collected data may be automatically compared to medical files, making it possible to alarm practitioner on pre-established criteria, which may save time for the practitioner, The drawback with telemedicine is however the limitation in capacity of the devices located at the patient. It is not possible to place very complex medical equipment at the location of the patient. This is both due to the cost of such equipment, but also due to the limited education of the patient. Thus, telemedicine is mainly useful for simpler equipment and predominantly for patient monitoring purposes.

In recent years also alternative medicine has had a steady growth. It is labeled "alternative'" because it is not always accepted by the mainstream health industry. It is often unregulated, discredited and also persecuted. One such example is the so called "Zapper", which is a pathogen killer that emits a waveform (frequency) into a subject. The "zipper" is a device developed and practiced by Hulda Dark, who claimed it as a panacea for exterminating parasites allegedly responsible for all illness, Clark was persecuted and discredited for claiming cure of her own, false diagnostics. The "Zapper" comprises a wave generator, which transmits electric energy waves into by patients in contact therewith.

There is a similar, but more sophisticated device, used by the Russian military, which generates ten different waveforms, allegedly destroying a variety of bacteria, microbes and parasites.

For over 50 years there have also existed bio-resonance medical devices for diagnostics and treatment. Bio-resonance devices are widely spread all over the world and for example ancient acupuncture evolved to electro-acupuncture, utilizes bio-resonance.

There are also more complex medical devices on the market today that combine wave-generating, bio-resonance etc. with computers and elaborate proprietary hardware and software to receive, to process and to transmit waveforms. There are devices that allegedly are capable to diagnose within seconds, the physiology of 72,000 points within a human body, far more sensitively than conventionally. There are also devices that claim treating a variety of physical and psychological conditions,

The success of such complex devices for treatments is largely dependable on the medical skills of the operator in order to make a correct interpretation of the detected waveforms and thereafter the application of a correct output, Although such devices may be portable they require the physical presence of a trained medical practitioner and his device in order to perform the medical examination.

### Summary of invention

Thus, there is a need for a device and a platform that, in a simple way and without the involvement of a practitioner, may be used to collect information about a patient and also is able to treat the patient. It would furthermore be advantageous if the device was able to measure electrical activity having another origin then a patient, for example animals, plants etc.

According to a first aspect of the invention a device is provided for collecting and transmitting electric signals from and to an end user. The device comprises a collector and a processing device connected to a communication network. The collector is configured to detect and collect data of electric signals emitted by the end user and send such data to the processing device, which is configured to process the data in such a way that sets of pertinent data characteristics are obtainable and then correlate, compare and analyze the pertinent data characteristics with corresponding data characteristics stored in a reference database available via the communication network. The processing device is further configured to, in response to the correlation, comparison and analyzing, receive data, via the communication network, for generation of biocompatible electrical spectra, which data is sent to the collector.

According to an embodiment of the present invention the collector is configured to detect and collect electric signals having an amplitude below 0,1 mV.

According to another embodiment of the present invention the collector has the form of a metallic or metal-coated conductor adapted to be hand-held by the end user or the form of at least one bracelet, whose interior is provided with a conductive, metallic surface and is adapted to be in contact with the skin of for example the ankles or wrists.

In another embodiment of the present, invention the collector comprises electronic circuitry and is configured to transmit and receive electric signals of very low amplitude, in the range of typically 0,0005- 0,1 mV, over a very wide range of frequencies, from 0,0005 Hz- 500 GHz.

In yet another embodiment the collector comprises transmitting antennas for producing a wave transmission that is able to electronically pulverize fungus or pest on infected crop.

In a further embodiment the processing device is configured to digitize and to filter out trivial information before the obtained characteristic is compared with the reference database.

According to a second aspect of the invention there is provided a platform for detecting and transmitting electric signals from and to an end user. The platform comprises the device for collecting and transmitting electric signals according to the first aspect, a control centre, service provider servers and a communication network for connecting the device for collecting electrical signals, the control centre and the service provider servers with each other. The control centre is configured to retrieve, transmit and control data from and to the collection and transmission device, the service provider server and the control centre itself.

According to a further embodiment the control centre is configured to perform various algorithmic manipulations of collected data and also to control and manage secure payment for any payable services and data provided.

### Brief description of drawings

The present invention will now be described in more detail in relation to the enclosed drawings, in which:
Figure 1 shows a schematic block diagram of the Nanoaura Platform,
Figure 2 shows a block diagram of the Collection and Transmission Domain,
Figure 3 shows an exemplary wavelet indicative of a certain type of virus, and
Figure 4 shows a flow chart of a method according to the present invention.

### Detailed description of different embodiments

Before different embodiments of the invention will be described a new term will be defined an introduced, namely the term "Nanoaura". Aura has been inherent to humanity for a very long time. In fact in most religions the head of Devine figures are depicted with a light (energy) band. Today there is technological evidence that meaningful signals of very low electrical nature, we may label it as aura, can be detected in any biological substance. By very low electrical nature is a meant a signal having an amplitude in the magnitude of µVolts up to mVolts. Such signals may at a first appearance look just like noise, but the initially chaotic pattern may be analyzed by using e.g. FFT(Fast Fourier Transform) or any other algorithmic processing in order to extract its distinct and precise characteristics, e.g. in the form of wavelets transformations. These very low electrical signals may be passive, i.e. only obtainable after and in response to, such as a resonant frequency wave, given to the biological substance, They may also be active signals, energy patterns or waves and any other type of non-cognitive expression from organic and inorganic matter or system. Such signals do also include signals that might become detectable in the future by new technologies having the capability of detecting and extracting meaningful information with higher accuracy then is possibly today. In context of the present invention all such signal emissions and its information contents will be grouped together and termed as Nanoaura.

Thus, Nanoaura defines all possible signals, which can be detected from any biological and non-biological substance. It may for example be at a nano-level of an individual atomic vibration or a more complex wave emission characteristic of an entire organism or the sub-system of the organism. Nanoaura may be active signals from natural emissions detected passively (non obtrusively) by sensors or passive Nanoaura signals, emitted after triggering and in response to the exposure containing resonant frequency signals etc. Artificial or technically produced Nanoaura may be labelled as a "pharmaceutical Nanoaura" developed in lieu of chemical pharmaceuticals. Such pharmaceutical Nanoaura may instruct electronic generation of waves in a biocompatible energy spectrum to users.

It should be further considered that it is not only possible to extract Nanoaura from a given subject, but that there are also a multitude of different Nanoaura types and levels within a given subject. This is best illustrated by the following example. From a human subject it is possible to simultaneously extract at least but not limited to the following Nanoauras: A number of relatively simple signals emitted from the resonant vibration of host microbes being subjected to resonant frequencies; a vast array of collective signals from about 80,000 diverse points of the human body; complex signals, which are the result of an advanced stage of abnormality or pathology within the bodily systems; energetic states signals and analysis and psychological state signals and analysis, It should be understood that the array of collective signals only has a meaning (of an existing or future pathology) once they are algorithmically processed, combined and compared with reference signals, to be described below.

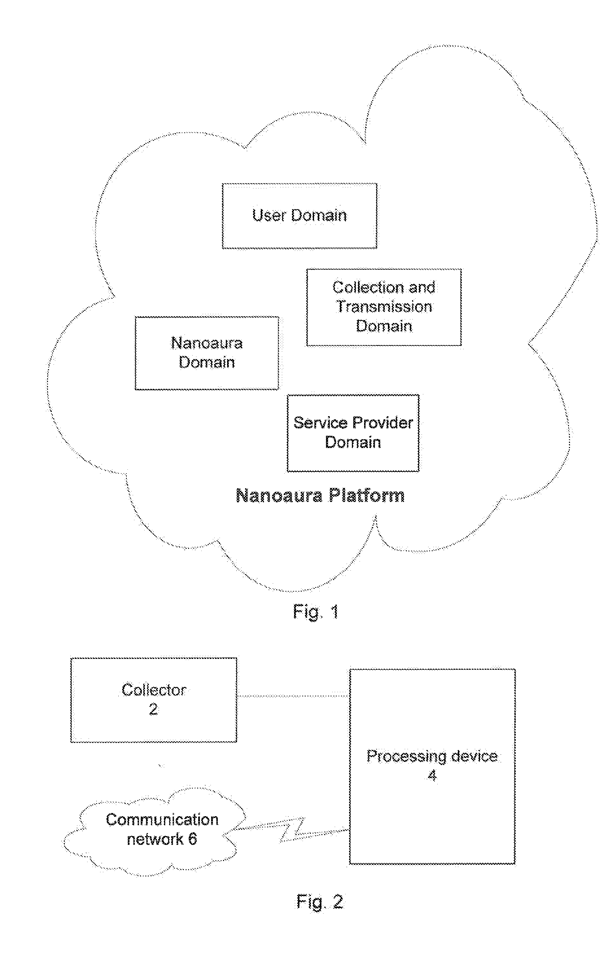

With reference to figure 1 a Nanoaura Platform (NP) will now be described. The NP comprises four different domains, namely a User Domain, a Collection and Transmission Domain, a Nanoaura Domain and a Service Providers Domain. As can be seen in figure 1 the different Domains are surround by a "cloud" in order to illustrate that they all may be interconnected via any communication network such as Internet, satellites or the like.

The User Domain defines the all possible end users of the NP. It is the Nanoaura of the end users which according to the present, invention is to be measured, collected and analyzed. End users are mainly human, but may also be animals, plants and loose microbes. End users may also be bacteria and parasites which may contaminate their hosts, for example a human being. Furthermore, air, water, etc may be an end user. Thus the end users is someone or something that interacts with the NP, via a vast array of methods, some of which will briefly outlined for illustrating the nature of end user and how they are connected to the NP.

One alternative to physically connect for example a person to the NP is to use some type of specialized collector, such as an electrode. The electrode may be a metallic or metal-coated conductor adapted to be hand-held by the person. Another possibility to connect a person to the NP is to use bracelets whose interior is provided with a conductive, metallic surface and is in contact with the skin offer example the ankles or wrists.

It is also possible to connect the end user contact-less by using a device comprising a transmitter and appropriate type of antennas connected thereto. In case the end user is a farmer and his pest-infected crop, an applicable device may be attached to the back of a tractor or a flying drone and connected to the NP platform in order to receive data information to be used to electronically pulverize the plantation or swamp. Such device may be a transmitter which will produce wave transmissions as instructed by the NP. In this particular case, with antennas, the outputted wave transmissions may be substantially higher then in the mVolt range in order to pulverize the swamp.

Now the Collection and Transmission Domain will be described in relation to figure 1 and 2. As is depicted in figure 2 this domain comprises a collector 2. Such device may be the electrode or the bracelets described above. In a preferred embodiment of the present invention the collector 2 is for example a reversible processing electrode part in form of an electric field sensor, consisting of a metal plate and electronic circuitry capable to transmit and receive electric signals of very low amplitude when in contact with for example a patient. With very low amplitude is meant an amplitude in the range of typically 0,0005- 0,1 mV. The electric field sensor may transmit and receive energy waves, i.e. low voltage electrical signals over a very wide range of frequencies, from 0,0005 Hz- 500 GHz. This field sensor or electrode may have any suitable form and is adapted to the specific application it will be used for. If the end user is a person the electrode may be a specific hand-held metallic piece possibly integrated as an attachment in a mobile phone, or as part of a steering wheel, a door handle etc as long as it is electrically conductible and has touchable points according to the NP application it will be used for.

While it is envisaged that a touchable, electrode part will be the most common and effective means of receiving and transmitting Nanoaura from and to an end user according to the present invention there will be other forms to receive and transmit Namoaura. Such forms include wireless transmitters and receivers, which may be used in treating, by resonance frequency techniques, malaria microbe on mosquito populations. Their swamp habitat may be subjected to electromagnetic waves by a transmitter, which carries the resonant frequency to destroy the malaria microbe. Such resonant frequency may be determined and downloaded from the Nanoaura Domain as will be described below. In a similar way farm plantation may be subjected to the appropriate frequencies in order to destroy pests and thereby avoiding pesticide chemicals. In the latter, the farmer will have connected his portable apparatus to the NP to determine the type of frequencies and/or wavelets useful for his plantation by exchanging information over the NP platform and finally downloading the applicable pesticide Nanoaura. Furthermore, using this apparatus as a remote Nanoaura receiver, the farmer will be able to take samples and by connecting and uploading such samples to the NP, find out the state of health of his plants.

Once again turning to figure 2 it may be seen that the collector 2 is connected to a processing device 4, which may comprise address and data busses, reversible DA- and AD-converters, controllers, software, filters, encoders and reversible transformer of dynamic feedback, which components all are well known to a person skilled in the art. The processing device 4 is used to process the electrical signal that has been detected and received by the collector 2 before it is sent away into the NP or the other way around is used to decode coded digital data received form the NP into the appropriate, biocompatible digital and/or analogue electrical waveforms which comprise the energy carrier wave modulated by the decoded signal and which is to be transmitted to the collector 2.

The processing device 4 has a multi-signal, multi-wave decoding and production capacity, all in one, as it may be producing/receiving resonant frequencies and/or wavelet transformations for transmission to the end user.

The processing device 4 exhibits intelligent and hardware connectivity to the NP for instance by connecting it to the Internet or other type of networks, and thus receives from and/or transmits to the NP by using the coded data described above.

The processing device 4 is further capable of conducting preliminary evaluations of collected Nanoaura, as digitized; to filter out trivial information from transmission, which will otherwise, overload the capacity of the communication channels unnecessarily. It does also encode/encrypt the meaningful, transmissible Nanoaura as per the coded protocols used by the NP.

Now the Nanoaura Domain will be described, which includes all the communication systems and devices, which allow Nanoaura data to be controlled and exchanged in a fully or semi automated way, the Nanoaura Domain may also be seen as a control centre. The major function of the Nanoaura Domain is to retrieve, transmit and control the data from and to the Collection and Transmission Domain and the Service Provider Domain. In the Nanoaura Domain data is also encoded, encrypted and stored. The Nanoaura Domain is also capable of performing various algorithmic manipulations of collected and newly produced Nanoauras, such as combinations, permutations, integration, differentiation and comparisons of the Nanoauros with reference data, Another major task of the Nanoaura Domain is to manage a reference database which contains Nanoaura (e.g. wavelet, transformation characteristics) suitably encoded and encrypted, allowing an automatic comparison of similarly collected and detected Nanoaura. Also other type of data such e.g. resonant frequency characteristics or energy characteristics may be stored in the reference database.

It should be understood that the Nanoaura Domain may be configured in numerous of ways in order to perform the above tasks. It is believed to within the capabilities of a person ordinary skilled in the art and is therefore not described in detail. Thus, it is for example believed that performing various algorithmic manipulations of Nanoauras is known to a person skilled in the art. The key to the invention is not the configuration of the NP as such but rather the use of and the creation of a reference data base making it possible to detect any imbalances at the User Domain,

The Nanoaura Domain also controls and regulates access to various levels of the NP, such as a public domain or the Service Provider Domain. Furthermore the Nanoaura Domain may control and manage secure payment for any payable services and data provided. The validation of Nanoauras and security against illicit and harmful interferences by "electronic drug dealers" who may attempt to supply electronic illicit substances via the NP or electronic drug piracy may also be controlled and managed by the Nanoaura Domain.

As is understood by a person skilled in the art the Nanoaura Domain comprises all the appropriate hardware, such as servers, clients, storage, etc. in order to execute proprietary and non-proprietary software in order to store and retrieve data. It is also configured with access to the Internet or any other form of widespread or local digital communication network.

The Service Provider Domain includes all the specially re-engineered or newly developed technologies and devices which allow professional service providers to develop products and services and to encode, encrypt and standardize these in order to deliver their practice and products to the NP.

Service providers may be but are not limited to scientific and academic institutions, government organizations of any relevant type such as customs, health and FDA-type regulators, WHO, private industrial health service providers, private practitioners such as doctors, specialists etc. NP will of course also be used by pharmaceutical companies as their distribution channel.

For pharmaceutical companies a whole new market will open. They may instead of their chemical molecules and formulae develop corresponding equivalent electronic formulae (Pharmaceutical Nanoaura). The coded digital formulas will be delivered through the NP and end up in a biocompatible, energy waveform and electronic signal which after clinical testing will be known to provide a specific cure. However, since the NP makes it possible to detect any imbalances of a user in an earlier stage than what is currently available there will be a market for a new generation of cures based on the detection of early stage nano-conditions of a user. If seen one by one such nano-condition variations may not mean much. However the ability to holistically detect ,combine and process tens of thousands of nano-conditions; most normal and some variant to normal, results in many meaningful combinations and permutations each able to divulge a possible forthcoming illness in a very early stage. Thus, "Pharmaceutical Nanoaura" will not only be electronic medicine in replacement of the chemical pharmaceuticals of today, but may also be used to suppress the root cause of illness and by restoring very early stage illness preconditions as well as cellular revitalization and re-energizing and thus providing for a healthier, longer life-span.

All the above described service providers will utilize standardized coding, encryption, processing, connectivity, software, and relevant NP provided technologies allowing their products and services to be compatible with the NP.

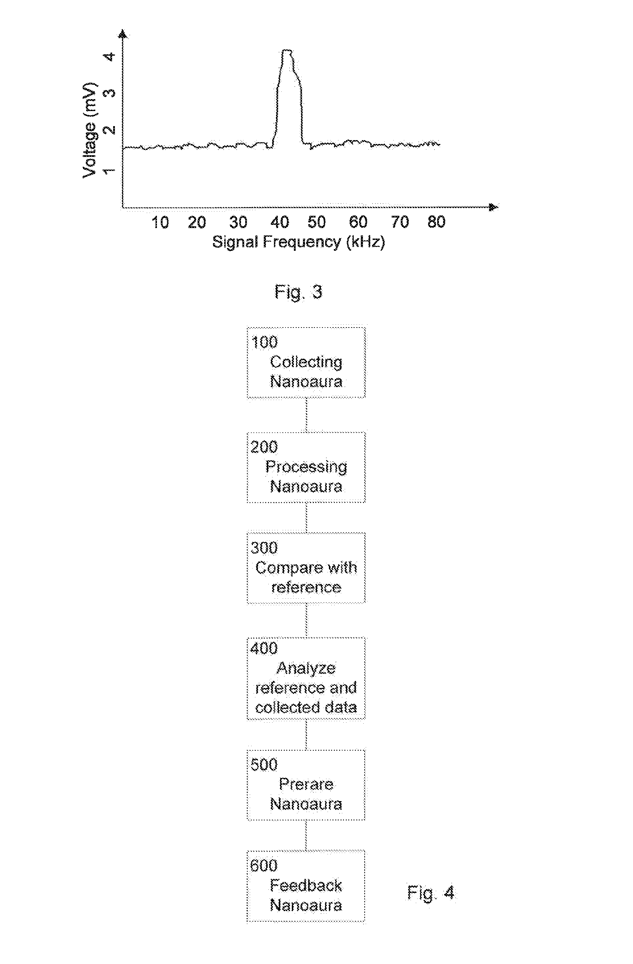

To better understand the present invention a method for how to use the NP will be described in conjunction with figure 4. This example will mainly describe the use of the NP when the end user is a patient. Thus in a first step 100 the patient will grab the collector 2 in form of an electrode with one or two hands. The collection of the Nanoaura may be done in at least two different ways depending on the information that is wanted. Firstly the electrode may act passive and only detect and measure the electric signal or signals present in the patient. Secondly the electrode may emit a low voltage stimulus over a wide frequency range and then detect its feedback. Thus when the data has been collected according to step 100, this data is sent to the processing device 4. As mentioned above the Nanoaura is collected from the patient in order to extract meaningful information there from.

Thus in the next step 200 the collected Nanoaura is mathematically processed by, e.g. computer processing of wavelets. By turning the collected Nanoaura into wavelets their patterns and behavior, i.e. the characteristic of the collected data, may render them identifiable and comparable with corresponding reference wavelets. An example of a wavelet is depicted in figure 3. As is evident the wavelet has a peak at a frequency somewhere between 40 and 50 kHz. This peak may correspond to a certain bacteria. Thus, in step 300 the obtained wavelet will be compared with wavelets stored in a reference database. Such a reference database is as mentioned above present in the Nanoaura Domain. This reference database contains all known digitized Nanoaura patterns, which describe characteristics of physiology of living species and electronic footprints of bacteria, virus etc. The NP is configured in such a way that Nanoaura from any subject can be automatically compared with reference Nanoaura available in the reference database and provides instant and automatic response thereto. For some complicated situations the automated process may not find a match in the reference database. This will evoke intervention of specialized medical service providers connected to the NP to assist in finding a match for the current Nanoaura.

When a match between the collected Nanoaura and the reference database is made the collected Nanoaura will by analyzed in step 400 together with the reference found in order to determine what imbalances the patient suffers. If an imbalance has been found, a Nanoaura cure will be prepared by looking for approved cures in a cure database. If a cure is found it will be sent back to the collector 2, which may feedback the Nanoaura cure that will remove the imbalance in step 600. Furthermore, custom-made Nanoaura cures may be devised and provided for by Nanoaura specialists, such as Doctors and equipment available through the Service Provider Domain.

There are many applications for the present invention. Below some examples thereof will be described. For example, diabetes patients may read their glucose level instantly and without invasiveness, by mere holding of a mobile phone equipped with an electrode as mentioned above and thereafter automatically log into their service providers to report the values.

The electrode at the end user may transmit Nanoaura that is especially designed against specific virus such as malaria or seasonal flu. By recognizing the Nanoaura footprint of a virus e.g. its resonant frequency or its wavelet characteristics, a diagnose and a possible cure may be initiated at an early stage. Today detection is made first after that symptoms are noticed by a person, i.e. post-incubation, In case of e.g. malaria treatment, NP substations in the Collection and Transmission Domain, containing the appropriate data and software can treat patients remotely, as far as the Internet, mobile telephony or satellite coverage can reach. Furthermore, it can be technically feasible to generate flux of energy emissions, at wavelengths that are destructive for the virus, thus eradicating malaria by apoptosis on mosquitoes.

Yet another application is to prevent pandemics. Once an epidemic or pandemic threatening virus is known, its Nanoaura footprint can be immediately loaded into the reference database for worldwide access. Before even developing the electronic formula for its eventual eradication by apoptosis, massive instant screening of travelers or anyone accessing a public place such as metro, train, plane, school, office etc. is possible by a mere simple touch of any metallic object designed to be the collector-electrode part of the NP. Alarm will be raised within a minute, while say a passenger is checking in for a flight or pushing a revolving barrier into a subway station, or even pushing the steel handle of a door into a public building.

NP also provides an alternative method of exterminating bacteria instead of using antibiotics. This is done by apoptosis caused by energy exhaustion through resonant vibration. In the foreseeable future biological substance such as a virus, bacteria and other parasites cannot escape this method of extermination based on precision quantum physics.

By using the NP it may also be easy to speed test athletes for doping. Thus, the Nanoaura of the athlete is detected, processed and then compared with doping Nanoauro footprints in a doping reference database. Thus, it is easy to test all athletes prior to or directly after the completion and to obtain the result immediately.

The inventors have already at this breakthrough stage achieved pathological and physiological treatments by using the NP components. This is done by feeding biocompatible energy spectra to patients, comprising a mix of energy and instructions to the brain having the appropriate chemical and electrical reactions in order to self generate the healing of the body internally.

One other major advantage of the NP technology is that diagnoses are made without the necessity of information exchange from patient. Indeed, a multi-level instant diagnosis enables correlation of psychosomatic syndromes and causes. By applying NP facilities through experienced neuron-psychiatrists, major advances in this sensitive medical sector may be expected.

As mentioned above the NP may not only be used for humans, but may also be apt for non-cognitive diagnostics for treating animals and plants.

The above mentioned and described embodiments are only given as examples and should not be limiting to the present invention. Other solutions, uses, objectives, and functions within the scope of the invention as claimed in the below described patent claims should be apparent for the person skilled in the art.

## Claims

1. A device for collecting and transmitting electric signals from and to an end user, comprising a collector (2) and a processing device (4) connected to a communication network (6), wherein the collector (2) is configured to detect and collect data of electric signals emitted by the end user and send such data to the processing device (4), which is configured to process the data in such a way that sets of pertinent data characteristics are obtainable and then correlate, compare and analyze the pertinent data characteristics with corresponding data characteristics stored in a reference database available via the communication network (6), the processing device (4) further being configured to, in response to the correlation, comparison and analyzing, receive data, via the communication network (6) for generation of biocompatible electrical spectra, which is sent to the collector (2).

2. A device according to claim 1, wherein the collector (2) is configured to detect and collect electric signals having an amplitude below 0,1 mV.

3. A device according to claim 1 or 2, wherein the collector (2) is a metallic or metal-coated conductor adapted to be hand-held by the end user.

4. A device according to claim 1 or 2, wherein the collector (2) is at least one bracelet, whose interior is provided with a conductive, metallic surface and is adapted to be in contact with the skin of for example the ankles or wrists of the end user.

5. A device according to any of the previous claims wherein the collector (2) comprises electronic circuitry and is configured to transmit and receive electric signals of very low amplitude, in the range of 0,0005- 0,1 mV, over a very wide range of frequencies, from 0,0005 Hz- 500 GHz.

6. A device according to any of claims 1, 2 or 5, wherein the collector (2) comprises transmitting antennas for producing a wave transmission that is able to electronically pulverize fungus or pest on infected crop.

7. A device according to any of previous claims, wherein the processing device (4) is configured to digitize and to filter out trivial information before the obtained characteristic is compared with the reference database.

8. A platform for detecting and transmitting electric signals from and to an end user, comprising the device for collecting and transmitting electric signals according to any of claims 1-7, a control centre, service provider servers and a communication network for connecting the device for collecting electrical signals, the control centre and the service provider servers with each other and wherein the control centre is configured to retrieve, transmit and control data from and to the collection and transmission device, the service provider server and the control centre itself.

9. A platform according to claim 8, wherein the control centre furthermore is configured to perform various algorithmic manipulations of collected data.

10. A platform according to claim 9, wherein the control centre furthermore is configured to control and manage secure payment for any payable services and data provided.
